(19) Europäisches Patentamt
European Patent Office
Office européen des brevets

(11) Publication number : **0 515 235 A2**

# (12) EUROPEAN PATENT APPLICATION

(21) Application number : **92304734.4**

(22) Date of filing : **26.05.92**

(51) Int. Cl.[5] : **C12N 15/46,** C12Q 1/68, A61K 39/15

A request for addition of accession number of the micro-organism on page 8 of the description has been filed pursuant to Rule 88 EPC. A decision on the request will be taken during the proceedings before the Examining Division (Guidelines for Examination in the EPO, A-V, 2.2).

(30) Priority : **23.05.91 GB 9111111**
**14.06.91 GB 9112875**

(43) Date of publication of application :
**25.11.92 Bulletin 92/48**

(84) Designated Contracting States :
**AT BE CH DE DK ES FR GB GR IT LI LU MC NL PT SE**

(71) Applicant : **NATURAL ENVIRONMENT RESEARCH COUNCIL**
**Polaris House North Star Avenue**
**Swindon SN2 1EU (GB)**

(71) Applicant : **INSTITUTO NACIONAL DE INVESTIGACIONS AGRARIAS**
**Embagjadores 68**
**E-28023 Madrid (ES)**

(72) Inventor : **Roy, Polly**
**8 Carey Close**
**Oxford, OX2 8HX (GB)**
Inventor : **Sanchez-Vizcaino Rodrique, Jose Manuel**
**c/o/Embagadores 68**
**E-28023 Madrid (ES)**

(74) Representative : **Ritter, Stephen David et al**
**Mathys & Squire 10 Fleet Street**
**London EC4Y 1AY (GB)**

## (54) DNA sequences coding for protein components of african horsesickness virus.

(57) DNA sequences coding for protein components of African horsesickness virus are provided, as well as the use thereof in the expression of polypeptides. The DNA sequences and expressed polypeptides are useful as diagnostic tools and in the case of the expressed polypeptides, as vaccine components. The DNA sequences of the invention were deririved by synthesising cDNA by reverse transcribing AHSV RNA, producing a library of cDNA clones from said synthesised cDNA, and screening said library for clones coding for full length AHSV proteins. The screening procedure included the step of identifying clones containing DNA inserts having the 3'-sequence CATTCAC and/or the 5′-sequence GTTAAAA.

EP 0 515 235 A2

This invention relates to DNA sequences coding for protein components of African horsesickness virus, to the use thereof in the expression of polypeptides, to the use of such DNA sequences and expressed polypeptides as diagnostic tools and in the case of the expressed polypeptides, as vaccine components.

African horsesickness (AHS) is an arthropod-borne disease of horses, caused by a double-stranded RNA orbivirus (family Reoviridae). In horses, infection is characterised by high mortality. The disease is endemic in Sub-Saharan Africa although occasional outbreaks have occurred in North Africa, the Middle East and in certain European countries (McIntosch, 1985). Recently (1988-1990) there have been several severe outbreaks of AHS in Morocco, Spain and Portugal. Although nine different serotypes of the virus (e.g. AHSV-1 to -9) have been identified in Africa (McIntosch, 1985; Howell 1962), only one serotype, AHSV-4, has been isolated in Spain and Portugal during the recent epidemics.

Little information is available on the genetics and molecular properties of AHSV. The virus is similar to the prototype orbivirus, bluetongue virus (BTV), both in its morphological structure and biochemical properties. Like BTV, AHS virions consist of seven structural proteins and ten double-stranded RNA segments (Oellermann et al, 1970; Bremer 1976). The outer capsid is composed of two major proteins (VP2 and VP5) while the inner capsid consists of 2 major (VP3 and VP7) and three minor proteins (VP1, VP4 and VP6). Using Northern blot hybridization studies involving cDNA clones and genomic RNA species, Bremer et al, have reported that the segment S7 RNA is highly conserved among the nine AHSV serotypes. However, no cross-hybridization was detected between AHSV and BTV serotypes.

In order to increase our understanding of the molecular biology and pathogenesis of AHSV and in particular in order to provide reagents for use in diagnostic kits and antigens for use as vaccine components it would be desirable to produce cDNA molecules coding for AHSV proteins.

However AHSV being an RNA virus, presents particular difficulties to researchers seeking to embark on its molecular analysis. In particular it is well known that the cloning of a full length DNA sequence corresponding to an RNA sequence coding for an orbivirus protein is extremely difficult. AHSV proteins are of high molecular weight and hitherto attempts to synthesis cDNA molecules coding for the entire polypeptide sequences have been unsuccessful and it has been possible to clone only relatively short stretches of the coding sequences. Attempts to join these small fragments have usually proved to be unsuccessful.

We have now surprisingly found that by employing scrupulous laboratory techniques and screening candidate clones for the presence of characteristic conserved sequences at the 3′ and/or 5′ ends of putative coding sequences, recombinant DNA molecules coding for the full length coding sequence of AHSV proteins can be isolated.

According to one aspect of the present invention, there are provided a recombinant DNA molecule comprising a DNA sequence coding for an AHSV protein.

The invention further provides a method for obtaining a cDNA clone coding for an AHSV protein which comprises synthesising cDNA by reverse transcribing AHSV RNA, producing a library of cDNA clones from said synthesised cDNA, and screening said library for clones coding for full length AHSV proteins, characterised in that the screening procedure includes the step of identifying clones containing DNA inserts having the 3′-sequence CATTCAC and/or the 5′-sequence GTTAAAA.

The DNA molecules provided according to the invention may be used in assay procedures for detecting AHSV RNA using known assay techniques. For example labelled probes may be produced from the the whole or portions of the DNA molecules of the invention and used in hybridization assays. Alternatively, the whole or portions of the DNA molecules may be used as primers in multiplication processes such as the polymerase chain reaction.

According to one specific embodiment of the invention, there is provided the complete sequence of the segment S7 that encodes the major core protein VP7 and document its homology to the VP7 of BTV.

The invention also provides a method of producing an AHSV protein which comprises in a suitable expression system the coding sequence of a recombinant DNA molecule as set forth above. One example of a suitable expression system comprises a recombinant baculovirus. In this embodiment, the AHSV coding sequence, operatively linked to a suitable promotor, for example the polyhedrin promotor, and incorporated into a baculovirus expression vector, is used to infect insects or insect cells. AHSV protein may the be isolated from the infected insects or cells. Other expression systems, for example systems based on yeast cells may also be used.

Polypeptides produced by expression of DNA coding sequences provided according to the invention may be used as reagents for detecting AHSV infections. Thus for example they may be used in immunological assays for detecting antibodies in AHSV-infected animals.

The polypeptides may also be used as vaccine components for eliciting a protctive affect in animals againsy AHSV infection.

The invention will now be described in more detail by way of example, with particular reference to the production of a DNA sequence corresponding to AHSV-4 VP7.

1. Viruses, cells and isolation of ds RNA

AHSV-4 (spanish isolate) was plaque-cloned using monolayers of BHK-21 cells. The viral dsRNA was purified (Ritter and Roy, 1989) and the 10 individual RNA segments were separated and isolated as described previously (Purdy et al, 1984; Purdy et al, 1985).

2. DNA cloning, sequencing

Polyadenylation of AHSV-4 dsRNA and synthesis of cDNA copies using an oligo$(dT)_{12-18}$ primer were undertaken as described elsewhere (Purdy et al, 1984; Purdy et al, 1985). The RNA templates were removed by treatment with 0.5M-KOH and dsDNA molecules were generated by self-annealing. The products were repaired using the Klenow large fragment of DNA polymerase, followed by 3′ tailing with dC and annealing to PstI-cut, dG-tailed, pBR322 plasmid DNA. After transformation, clones containing the viral sequences were recovered and screened by colony hybridization (Grunstein and Hogness, 1975) HinfI restriction patterns of the recombinant plasmids were compared as described previously (Purdy et al, 1984; Purdy et al, 1985). The sequences of DNA clones were mainly determined by the dideoxynucleotide sequencing method (sanger et al, 1977) using appropriate DNA fragments subcloned into M13 vectors (Oldfield et al, 1990). The terminal sequences of the clones were determined by the method of Maxam and Gilbert (Maxam and Gilbert, 1980).

3. The complete nucleotide sequence of AHSV RNA segment 7

Several AHSV S7 cDNA clones were identified by colony hybridization and Northern blot analyses (Grunstein and Hogness, 1975). Several positive clones were then selected for further analyses. The inserts were excised from pBR322 with PstI and in order to determine the sizes of these inserts they were analysed by restriction enzyme digestion. Two clones appeared to be full-length. One of them (A) was selected for sequence analyses. To determine if the clone is indeed full length the terminal sequences were determined by the Maxam and Gilbert method (Maxam and Gilbert, 1980) as described previously (Purdy et al, 1984) while the remaining sequences of the clone were determined by the didoxy method (Sanger et al, 1977). The 5′ and 3′ termini of the clone were found to contain the characteristic terminal sequences of orbivirus RNA segments (Rao et al, 1983; Mertens and Sanger, 1985; Wilson et al, 1990), namely 5′ GTTAAA... and ...ACTTAC 3′ termini (Fig. 1), indicating that the AHSV VP7 clone was full-length.

The complete nucleotide sequence of the cDNA of S7 in its coding (mRNA) sense is presented in Fig. 1, with the predicted amino acid sequence of the single long open reading frame shown above the cDNA. The S7 segment is 1179 base pairs long. The Mr value of the dsRNA is calculated to be $7.0 \times 10^5$. The coding strand of the AHSV-4 S7 RNA has a calculated base composition of 27.5% U, 26.4% A, 19.7% C, and 26.5% G, ie., comparable to the base composition of the RNA sequent S7 of BTV-10 (Yu et al, 1988). The S7 mRNA strand of AHSV-4 has a long 3′ non-coding sequence, also like that of the S7 RNA of BTV-10 (100-110 nucleotides). The 5′ terminal non coding sequence of the S7 RNA of both viruses is much shorter (17 nucleotides).

The 5′ and 3′ termini of AHSV S7 show partial inverted complementarity and capable of forming secondary structures as shown in Fig 2a for the message sense cDNA sequence. Similar complementary sequences were identified from the previously published sequence data of the S7 species of BTV-10, BTV-13 and BTV-17 (Yu et al, 1988; Kowalik and Li, 1989; Kowalik et al, 1990, see Fig. 2b,c,d). Such secondary structures, albeit of different lengths and composition, are common to all the RNA segments of BTV (and epizootic haemorrhagic disease virus, EHDV) that have been determined so far. Interestingly in the proposed structures there are some common features, for example, there is a looped out sequence (CACA for the S7 cDNAs shown in Fig. 2), proximal to the 3′ terminus. Also the 3′ and 5′ ends have the potential for two alternative forms, either that shown in Fig. 2, or with a 5′ overhang (G) when the third 5′ nucleotide (T) pairs with the second 3′ nucleotide (A). Noteworthy, too, is the fact that the translation termination codon for the VP7 protein of AHSV (TAG, underlined in Fig. 2) is located within a potentially base-paired structure, although for BTV S7 it is upstream of a similar structure. The AHSV VP7 translation initiation codon (ATG, underlined, Fig. 2) is also located within a potentially base-paired structure.

The observation that the single-stranded RNA's have the potential of forming secondary structures is presumably not coincidental. Although the viral RNAs are considered to be double-stranded in the virions, it is possible that the RNA sqequences are held by the viral proteins (at least temporally) in alternative configurations to facilitate transcription by the virion RNA polymerase. Secondary structures may also play a role in protecting mRNA from exonuclease digestion or, for the 3′ proximal region, in facilitating translation termination. They may also be involved in segregating the 10 mRNA species for encapsidation within subcore particles that are progenitors of virions (possibly in association with the NS2 phosphoprotein, or other viral components), (Roy, 1989; Thomas et al., 1990). In this context it is noteworthy that the 3'- and 5'- terminal sequences of influenza virus showed similar inverted complementarity (Desselberger et al, 1980).

Hsu et al, (1987) recently have demonstrated that within the virus particle, the termini of influenza viral RNA genome exists in a panhandle configuration and protein-RNA interactions are required to stabilize these structures. In wound tumor viral RNA similar inverted repeats within the terminal regions were identified (Anzola et al, 1987). These termini were shown to form intramolecular structures in the absence of protein factors. It was postulated that these configuration subsequently stabilized by protein-RNA interaction leading to replication and packaging (Xu et al, 1989).

4. Characteristics of the protein encoded by the S7 of AHSV-4 and comparison with the VP7 of BTV

The single major open reading frame of the S7 RNA of AHSV predicts a polypeptide of 354 amino acids, five amino acids larger than the VP7 of BTV-10 (Yu et al, 1987). The molecular size ($M_r$) of VP7 is estimated to be 38,107, slightly smaller than the VP7 of BTV (38,545).

The AHSV-4 VP7 protein has a low content of charged amino acids (ie. R + K +1/2H = 25.5; D + E = 27), comparable to the BTV-10 VP7 protein (ie., R + K + 1/2H = 28; D + 3 = 27). Both proteins are rich in hydrophobic amino acids, particularly alanine, methionine and proline. Sequence alignment indicates that the VP7 proteins are in fact homologous, as shown in Fig. 3. The close relationship between the two proteins is indicated by the fact that only 4 gaps are required for the diagon alignment (data not shown). Although 44% of the amino acids residues at similar sites were homologous, when amino acids of similar character were considered, the homology corresponded to some 70%. The distribution of similar sequences was not evenly spread throughout the molecule. When the hydropathic profiles of the VP7 protein of AHSV-4 were compared with those of BTV-10, there were some similarities (Fig. 4) although two proteins have very distinct profiles. Both share strong homology in their amino termini profile (yp to 100 Nindine) and some homology in the carboxyl termini. However the middle regions appeared to be much more divergent.

Additional disclosure relating to the invention, particularly the expression of AHSV VP7 in a baculovirus expression system based upon A californica nuclear polyhedrosis virus is given in Appendix A appearing in the following pages 9 - 15 and in the additional sheets of drawings marked "Appendix A - Figures"

Plasmid pACYMI-AHSV-4 has been deposited at NCIMB on 17th May 1991 under Accession No. NCIMB 40420.

Recombinant baculovirus AcAHSV-4.7 has been deposited at ECACC on 22nd May 1992 under Accession No.

# APPENDIX A

African horsesickness which is endemic predominantly in sub-Saharan Africa, a gnat (*Culicoides*) transmitted disease of horse, mules and donkeys. In severe cases death occurs from pulmunary oedema. Goats, zebras, ferrets and dogs are also susceptible. The etiological agent is an Orbivirus within the Reoviridae family. Like the bluetongue orbivirus (BTV), the double-stranded RNA genome of African horsesickness virus (AHSV) consists of ten segments located within an icosahedral core particle. The cores of the orbiviruses are composed of two major (VP3, VP7) and three minor proteins (VP1, VP4, VP6). The core is surrounded by two other proteins (VP2 and VP5) forming a loosely bound capsid. Unlike BTV, the molecular biology of AHSV is in its infancy. However, recently several outbreaks of the disease in Southern Europe have caused some alarm around the world.

In order to develop suitable diagnostic reagents and strategies to control the disease, we have begun to investigate the structure-function of the various genes and gene products of AHSV. Recently we have reported the complete sequence of the major core protein, VP7 of serotype 4(isolated from Spain). In this report we describe the construction and isolation of a recombinant baculovirus that synthesizes the VP7 of AHSV-4 in infected *Spodoptera frugiperda* cells. The strong polyhedrin promoter of *Autographa californica* nuclear polyhedrosis baculovirus (AcNPV) vector was used for the high level expression of the VP7. The synthesized protein was characterized, and the biologically active form of VP7 was purified. The ability of the purified VP7 protein for diagnosis of nine AHSV serotypes was then demonstrated using an enzyme-linked immunoabsorbant assay (ELISA).

## MATERIALS AND METHODS

*Virus and cells. S. frugiperda* cells were grown in suspension or monolayer cultures at 28°C in TC100 medium supplemented with 10% foetal calf serum. AcNPV and recombinant baculoviruses were plaque purified and propagated as described by Brown and Faulkner, 1977.

*Preparation of transfer vector.* Standard DNA manipulation techniques were used to construct recombinant transfer vectors (Maniatis *et al.*, 1982). Restriction enzymes, T4 DNA ligase and the Klenow large fragment of DNA polymerase were purchased from Amersham International (Amersham, UK). Calf intestinal alkaline phosphatase was obtained from Boehringer Mannheim (Mannheim, FRG).

APPENDIX A

The construction of plasmid pAcYM1/AHSV4.7 is summarised in Figure 1. The full length cDNA representing AHSV-4 dsRNA segment 7 (S7) was released from pBR322 cloning vector by *PstI* digestion and the homopolymeric tails that had been added in the original cloning procedure were removed by digestion with *Bal*31 (Roy *et al.*, 1991). The products were then repaired using the Klenow fragment of DNA polymerase and inserted into pUC4K that had been previously digested with *SalI*, repaired and dephosphorylated as shown in Figure 1. The terminal sequences of candidate inserts were sequenced according to Maxam and Gilbert (1980) and a clone retaining the entire coding region as well as 7 base pair upstream of ATG start codon was selected and ligated into the *BamHI* site of the baculovirus transfer vector pAcYM1 (Matsuura *et al.*, 1987). The derived recombinant transfer vectors characterized by restriction enzyme and sequence analyses. One of the recombinant vector (pAcYM1/AHSV4.7) contained the AHSV S7 gene insert in the correct orientation for expression directed by the AcNPV polyhedrin promoter.

*Construction of the recombinant baculovirus.* The transfer vector (pAcYM1/AHSV4 VP7) was used to generate recombinant baculovirus through transfection and homologous recombination as previously described. Co-transfection was performed by the lipofectin method as follows. The transfection mixture (24µl containing 1µg of pAcYM1/AHSV4.7 DNA, 50ng of baculovirus DNA and 8µl of lipofectin) was incubated for 15 min at RT. The mixture was inoculated on $1.5 \times 10^6$ cells of S.f cells. The supernatants of the infected cells were harvested at 2 days post-infection and the recombinants were selected as described previously (Matsuura *et al.*, 1987).

*Protein analyses.* S.f cells in 35mm tissue culture dishes were infected with the virus at a multiplicity of 10PFU per cell, and the cells were incubated at 28°C. At the end of the infection the cells were rinsed 3 times with phosphate-buffered saline (PBS) and the cells lysed in 150µl of RIPA buffer (1% Triton X-100, 1% sodium deoxycholate, 0.5M NaCl, 0.05M Tris-HCl pH 7.4, 0.01M EDTA, 0.1% SDS). Aliquots of the protein samples were boiled for 10 min in dissociation buffer (2.3% SDS, 10% Glycerol, 5% (v/v) β-meracptoethanol, 62.5mM Tris-HCl (pH 6.8) 0.01% BPB). Proteins were analysed by a 10% polyacrylamide gel electrophoresis in the presence of SDS (Laemmli, 1970).

After the electrophoresis, the proteins were either stained with Kenacid blue (Overton *et al.*, 1987) or blotted onto Immobilon membrane using a semi-dry electroblotter. The blot was incubated for 1h in the blocking solution (5% w/v) milk powder and 0.1% NP40 in PBS), then transferred to a blocking solution

APPENDIX A

containing AHSV-4 polyclonal antisera (1:1000 dilution), and incubated for 1h. After 3 wahses for 10 min in the same buffer, the bound antibody was detected using an appropriate alkaline phosphatase conjugated IgG (Sigma Chemicals, St. Louis, MO), BCIP and NBT (Gibco, BRL International) were used as substrates for the alkaline phosphatase.

*Purification of AHSV expressed VP7 from S. frugiperda cells.* AHSV-4.7 infected *S. frugiperda* cells (2.$10^9$ cells) were harvested 4 days post-infection by centrifugation at 3000 rpm for 15 min at 4°C and rinsed in PBS. Cell pellets were resuspended in 24ml 10mM Tris-HCl (pH 7.5) 150mM NaCl containing 0.5% Nonidet P40 and left on ice for 15 min. The treated cells were then recentrifuged at 1000 rpm for 10 min to remove cell debris. The supernatant was loaded on a 30% to 80% (w/v, 10ml) continuous sucrose gradient in 10mM Tris-HCl (pH 7.5). The gradients were centrifuged at 26,000 rpm for 2 h at 4°C in a SW41 rotor (Beckman), and Fractions were collected from the gradients, samples containing the AHSV-VP7 were pooledand dialysed overnight at 4°C in 10mM Tris (pH 7.5). Insoluble material was removed by low-speed centrifugation and the supernatant was freeze-dried and stored at -20°C.

*ELISA test using purified VP7.* Each well of a 96 well PVC microtitre plate (Flow Laboratories) was coated overnight at 4°C with 50µl VP7 (i.e., approximately 100ng per well) in 0.1M sodium bicarbonate, pH 9.5, After adsorption of the antigen, the plates were saturated in blocking buffer (5% skimmed milk powder in TBS (144mM NaCl in 25mM Tris-HCl (pH 7.6)) for 30 min at room temperature. After three washes in blocking buffer, the plates were incubated with 200µl/well of serial dilutions of reference anti-AHSV-4 horse serum in blocking buffer for 1h at room temperature. After three washes in the same buffer, the plates were incubated with 200µl/well of 1:1000 dilution of anti-horse or anti-rabbit IgG-phosphatase alkaline conjugate (Sigma) for 1h at room temperature. Following three washes in TBS alone, the enzyme-linked secondary antibodies were detected using p-nitrophenyl phosphate disodium substrate (Sigma) in 1M Tris pH 8.0. The absorbance at 405nm was measured using a multichannel spectrophotometer.

## RESULTS

*Construction and isolation of a recombinant baculovirus (Figure 1).* A recombinant baculovirus containing the AHSV-4 segment 7 sequence was constructed as described under Methods. To facilitate the cloning representing cDNA of RNA segment 7 (S7) into pBR322, homopolymeric tails had been added (Roy *et al.*, 1991). These sequences were removed by digestion with the

APPENDIX A

exonuclease *Bal*31. The DNA copy containing the entire coding region of S7 gene was therefore inserted into the *BamHI* site of this vector and the recombinant transfer vector AHSV-4.7 was identified. Subsequently, recombinant viruses (e.g., AcAHSV-4.7) were obtained by transfecting *S. frugiperda* cells with a mixture of this plasmid DNA and infectious wild-type AcNPV DNA in the presence of lipofectin as described in Methods. The presence of the S7 gene in one of the recombinant viruses (AHSV-4.7), was confirmed by subjecting the viral DNA to Southern analyses as described previously (Inumaru and Roy, 1987; data not shown).

*Expression of AHSV-4 major core protein VP7 in S. frugiperda cells.* To demonstrate that the VP7 of AHSV-4 was synthesized in cells infected with the recombinant baculovirus AHSV-4.7, samples of extracted protein were analysed by SDS-PAGE together with samples of mock-infected and wild-type AcNPV-infected cells and the protein bands were detected by staining with Kenacid blue as described in Methods.

As shown in Figure 2, cells infected by AcAHSV-4.7 synthesized a major 38KDa protein, i.e., similar to the predicted from the amino acid sequence of the segment 7 gene product (Roy *et al.*, 1991), and equivalent to the recombinant BTV-10 VP7 protein (Oldfield *et al.,* 1991). The equivalent protein band was not present either in mock-infected cells or in cells infected with wild type AcNPV. The maximum expression of VP7 was obtained after 4 days post-infection and the expression level of the VP7 protein was estimated to approximately 100mg/litre of infected cells ($2 \times 10^9$ cells). Since the level of VP7 expression was very high, it was of interest to examine whether the recombinant virus infected cells showed any visible protein mass. As shown in Figure 3, infected cells when examined under light microscope, contained distinct pin-shaped crystal-like structure in the cytoplasm. Both infected and AcNPV infected cells lack such protein aggregates. The structure exhibited variations both in size and number in each cell each containing one to three such crystals. It is noteworthy in this context that the similar structure can be seen in the BHK-21 cells infected with AHSV virus (personal communication, Dr Peter Mertens).

To confirm that the 38kDa protein was indeed a AHSV-4 gene product, a duplicate gel was subjected to Western immunoblot analysis using polyclonal anti-AHSV-4 horse antiserum as described in Methods. The positive signals obtained confirmed that the expressed 38kDa band was an authentic AHSV gene product (Figure 4).

# APPENDIX A

*Purification of AHSV-VP7 protein produced in insect cells.* In order to use the expressed AHSV-VP7 protein as a source of antigen for diagnosis, a purification procedure was developed. A 1l spinner culture of $2.10^9$ cells was infected with the recombinant baculovirus AcAHSV-4.7. The cells were harvested at the maximum VP7 protein expression and lysed in the presence of NP40 detergent, after pelleting the cell debris the expressed VP7 was found to be present in the cytoplasmic extract of *S. frugiperda* cells infected with AcAHSV-4.7 (Figure 5). After subsequent centrifugation on a 30% to 80% sucrose gradient the VP7 protein was localized in fractions 9 to 11 (1ml fractions) at the bottom of the 12ml gradient with a peak in fraction 10. This material consisted of 90% pure VP7 as shown in Figure 5. The yield was determined to represent some 15mg/l purified VP7 protein.

*ELISA test.* VP7 has been identified as a group specific antigen for BTV, the Orbivirus prototype (Huismans and Erasmus, 1981; Gumm and Newman, 1982; Hübschle and Jang, 1983; Oldfield *et al.*, 1990). To determine whether AHSV-VP7 was similarly the group-specific antigen for the nine AHSV serotypes (1-9) and to determine the efficacy of the purified VP7 as a diagnostic reagent, purified VP7 was coated onto microwell plates and tested by ELISA for the presence of anti-VP7 antibodies in polyclonal antisera representing 9 AHSV serotypes (AHSV-1 to 9). Serial dilutions of each serum were tested to determine the VP7 antibody titers defined as dilution of sera giving 50% of the maximum absorbance (Table 1). Semi-purified AHSV-4 virus was included for comparison in the test. Figure 6A shows the results of the analyses as the maximum absorbance at 600nm. The recombinant AHSV-4 VP7 was recognized by all the AHSV antisera tested in ELISA. The results were comparable to those obtained with semi-purified AHSV-4 virus for low titer sera (i.e., serotype 1, 2, 3, 4 and 9). For the high-titer sera (i.e., serotype 6, 7 and 8) the maximum absorbance was lower with the recombinant AHSV VP7 probably due to less amount of protein used thanwith the semi-purified virus.

To investigate further the potentialities of the recombinant AHSV-VP7 for diagnostic purposes, sera from infected animals or from animals vaccinated with either monovalent (10 samples), or polyvalent vaccines (18 samples) were also tested in the same conditions. Sera from non-immune animals were included as controls. Figure 6B presents the results as the mean value between the different sera and shows that the recombinant AHSV-VP7 reacted as well as semi-purified virus with the different antisera. With the negative sera, slightly higher background was detected with the recombinant AHSV-VP7 in these conditions.

A P P E N D I X   A

## FIGURE LEGENDS

Figure 1. Diagnammatic representation of the construction of the transfer vector pAcYM1/AHSV-4.7 as described in Methods. The sequence of the 5' insertion site is shown with the initiation ATG of segment 7 underlined.

Figure 2. Expression of VP7 in *S. frugiperda* cells by a recombinant baculovirus AcAHSV-4.7. Protein samples were resolved on a 10% SDS-polyacrylamide gel and stained with Kenacid blue. Lanes: 1) Molecular weight markers; 2) Mock-infected *S. frugiperda* cell lysate; 3) AcNPV infected *S. frugiperda* cell lysate, 3a) 24h, 3b) 48h, 3c) 72h, 3d) 96h post-infected; 4) AcAHSV-4.7 infected *S. frugiperda* cell lysate, 4a) 74h, 4b) 48h, 4c) 72h, 4d) 96h post infection.

Figure 3. A. AcNPV infected S. frugiperda cells (40 × magnification). B. AcAHSV-4.7 infected S. frugiperda cells (40 × magnification).

Figure 4. Western Blot analysis of the samples from figure 2 using anti-AHSV-4 polyclonal serum. Lanes: 1) AcNPV infected *S. frugiperda* cell lysate, 2) AcAHSV-4.7 infected *S. frugiperda* cell lysate.

Figure 5. Samples containing VP7 at different stages of purification were analysed by SDS-PAGE and stained with Kenacid blue. Lnaes: 1) cytoplasmic extract of *S. frugiperda* cells infected with AcAHSV-4.7. 2) VP7 after centrifugation on a 30% to 80% linear sucrose gradient and dialysis in 10M of Tris-HCl pH 7.4; M, Molecular weight markers.

Figure 6. ELISA with purified AHSV antigen. Different amount of purified AAHSV-VP7 protein (25ng to 500ng) were bound to the solid phase, serial dilutions of a anti-AHSV-4 polyclonal reference serum (1/100 to 1/100,000) were tested. The reactivity of the serum withthe different concentration of antigen is expressed by a absorbance at 405nm as a function of the serum dilution.

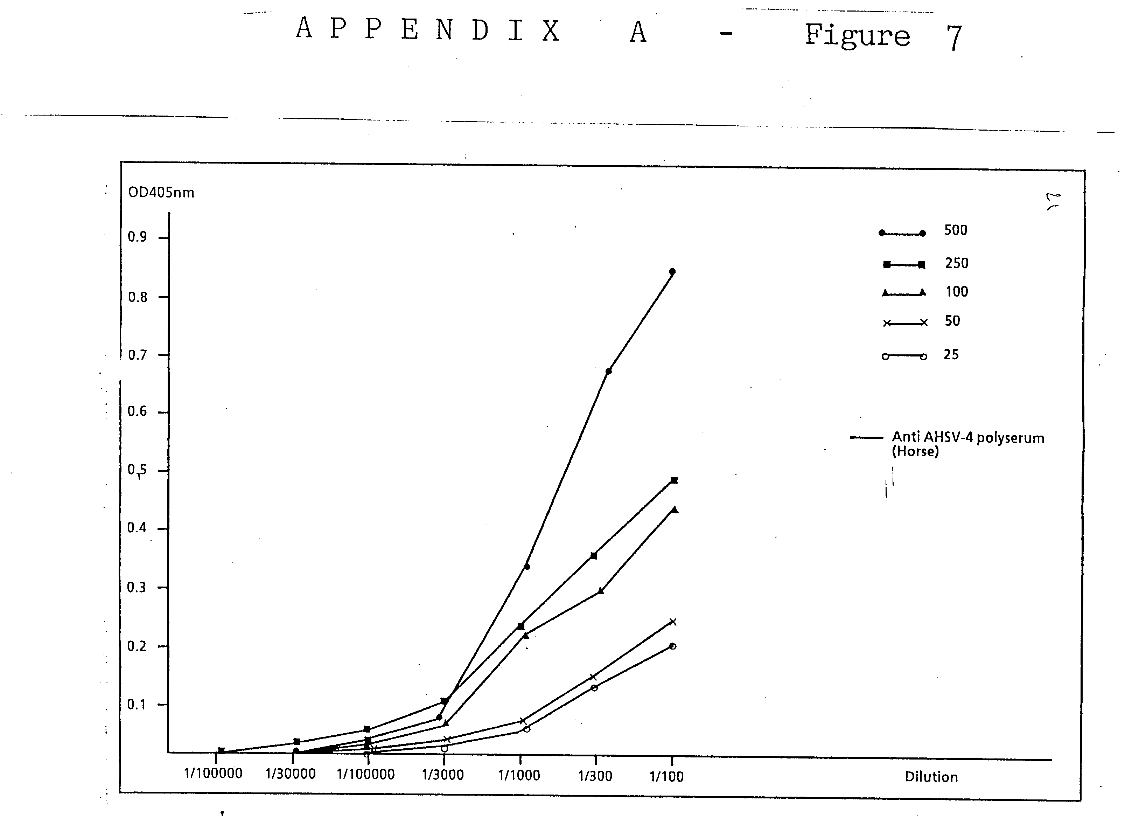

Figure 7. ELISA with AHSV antigen - serial dilutions

APPENDIX A

## REFERENCES

Brown, M. and Faulkner, P. (1977). A plaque assay for nuclear polyhedrosis viruses using a solid overlay. Journal of General Virology **36**: 361-364.

Huismans, H. and Erasmus, B.J. (1981). Identification of the serotype-specific and group-specific antigens of bluetongue virus. Onderstepoort Journal of Veterinary Research **48**: 51-58

Inumaru, S. and Roy, P. (1987). Production and characterization of the neutralization antigenic VP2 of bluetongue virus serotype 10 using a baculovirus expression vector. Virology **157**: 472-479.

Laemmli, U.K. (1970). Cleavage of the structural protein during assembly of the head of bacteriophage T4. Nature **227**: 680-685

Maniatis, T., Fritsch, E.F. and Sambrook, J. (1982). Molecular cloning: A Laboratory Manual. Cold Spring Harbor Laboratory, Cold Spring Harbor, N.Y., USA.

Matsuura, Y., Possee, R.D., Overton, H.A. and Bishop, D.H.L. (1987). Baculovirus expression vectors: The requirements for high level expression of proteins including glycoproteins. Journal of General Virology **68**: 1233-1250.

Maxam, A. and Gilbert, W. (1980). Sequencing end-labelled DNA with base-specific cleavages. Methods in Enzymology **65**: 499-560.

Oldfield, S., A. Adachi, T. Urakawa, and P. Roy. (1990). Purification and characterization of the major group-specific core antigen VP7 of bluetongue virus synthesized by a recombinant baculovirus. Journal of General Virology **71**: 2649-2656.

Overton, H.A., T., Ihara, and D.H.L. Bishop. (1987). Identification of the N and NSs proteins coded by the ambisense sRNA of Punta Toro phlebovirus using monospecific antisera raised to baculovirus expressed N and NSs proteins. Virology **157**: 338-350.

Hirasawa, T., Fernandez, M. and Roy. P. (1991). Sequence analysis of major capsid protein VP7 of African Horsesickness virus serotype 4 (recently isolated from Spain) reveals an unexpected close relationship with bluetongue virus. Journal of General Virology (in press).

## Claims

1. A recombinant DNA molecule comprising a DNA sequence coding for an AHSV protein.

2. A recombinant DNA molecule according to Claim 1 coding for AHSV protein VP7.

3. A recombinant DNA molecule according to Claim 2 comprising the DNA coding sequence depicted in Fig. 1.

4. A method for obtaining a cDNA clone coding for an AHSV protein which comprises synthesising cDNA by reverse transcribing AHSV RNA, producing a library of cDNA clones from said synthesised cDNA, and screening said library for clones coding for full length AHSV proteins, characterised in that the screening procedure includes the step of identifying clones containing DNA inserts having the 3′-sequence CATTCAC and/or the 5′-sequence GTTAAAA.

5. A recombinant DNA molecule comprising a DNA sequence coding for an AHSV protein, whenever obtained by a method according to Claim 4.

6. The use of a probe selected from the following:
   (i) a probe comprising a recombinant DNA molecule as claimed in any of claims 1 to 3 and 5
   (ii) a probe comprising a recombinant DNA molecule capable of hybridising to a recombinant DNA molecule as claimed in any of claims 1 to 3 and 5 for detecting AHSV.

7. A method of producing an AHSV protein which comprises in a suitable expression system the coding sequence of a recombinant DNA molecule as claimed in any of claims 1 to 3 and 5

8. A method according to Claim 7, wherein said expression system comprises a recombinant baculovirus.

9. A polypeptides produced by the method of any of Claim 7 or claim 8.

10. The use of a polypeptide according to claim 9 as a reagent for use in an assay method for detecting AHSV infections.

11. The use of a polypeptide according to claim 9 as a vaccine components for eliciting a protective affect in animals against AHSV infection.

```
                  M  D  A  I  A  A  R  A  L  S  V  V  R  A  C  V  T  V  T  D  A  R  V  S  L  D  P  G  V  M  E  T  L  G  I
GTTAAAATTCGGTTAGGATGGACGCGATAGCAGCAAGAGCCTTGTCCGTTGTACGGGCATGCGTCACAGTGACAGATGCGAGAGTTAGCTTGGATCCAGGAGTGATGGAGACGTTAGGGA
        10        20        30        40        50        60        70        80        90       100       110       120

 A  I  N  R  Y  N  G  L  T  N  H  S  V  S  M  R  P  Q  T  Q  A  E  R  N  E  M  F  F  M  C  T  D  M  V  L  A  A  L  N  V
TTGCAATTAATAGGTACAATGGTTTAACGAATCATTCGGTATCGATGAGGCCACAAACCCAAGCAGAACGAAATGAAATGTTTTTTATGTGTACTGATATGGTTTTAGCGGCATTGAACG
       130       140       150       160       170       180       190       200       210       220       230       240

 Q  I  G  N  I  S  P  D  Y  D  Q  A  L  A  T  V  G  A  L  A  T  T  E  I  P  Y  N  V  Q  A  M  N  D  I  V  R  I  T  G  Q
TCCAAATTGGGAATATTTCACCAGATTATGATCAGGCGTTGGCAACTGTGGGAGCTCTTGCAACGACTGAAATTCCATATAATGTTCAGGCCATGAATGACATCGTTAGAATAACGGGTC
       250       260       270       280       290       300       310       320       330       340       350       360

 M  Q  T  F  G  P  S  K  V  Q  T  G  P  Y  A  G  A  V  E  V  Q  Q  S  G  R  Y  Y  V  P  Q  G  R  T  R  G  G  Y  I  N  S
AAATGCAAACATTCGGACCAAGCAAAGTACAGACGGGACCTTATGCAGGAGCAGTTGAGGTGCAACAATCTGGCAGATATTACGTACCGCAAGGTCGAACACGTGGTGGGTACATTAATT
       370       380       390       400       410       420       430       440       450       460       470       480

 N  I  A  E  V  C  M  D  A  G  A  A  G  Q  V  N  A  L  L  A  P  R  R  G  D  A  V  M  I  Y  F  V  W  R  P  L  R  I  F  C
CAAATATTGCTGAAGTGTGTATGGATGCAGGCGCTGCGGGACAGGTCAATGCGCTGCTAGCCCCAAGGAGGGGGGACGCAGTCATGATCTATTTTGTTTGGAGACCATTGCGTATATTTT
       490       500       510       520       530       540       550       560       570       580       590       600

 D  P  Q  G  A  S  L  E  S  A  P  G  A  P  G  T  F  V  T  V  D  G  V  N  V  A  A  G  D  V  V  A  W  N  T  I  A  P  V  N
GTGATCCTCAAGGTGCGTCACTTGAAAGCGCTCCAGGGGCTCCAGGGACTTTTGTCACCGTTGATGGAGTAAATGTTGCGGCTGGAGATGTCGTCGCATGGAATACCATTGCACCAGTGA
       610       620       630       640       650       660       670       680       690       700       710       720

 V  G  N  P  G  A  R  R  S  I  L  Q  F  E  V  L  W  Y  T  S  L  D  R  S  L  D  T  V  P  E  L  A  P  T  L  T  R  C  Y  A
ATGTTGGAAATCCTGGGGCACGCAGATCAATTTTACAGTTTGAAGTGTTATGGTATACGTCTTTGGATAGATCGCTAGACACGGTTCCGGAATTGGCTCCAACGCTCACAAGATGTTATG
       730       740       750       760       770       780       790       800       810       820       830       840

 Y  V  S  P  T  W  H  A  L  R  A  V  I  F  Q  Q  M  N  M  Q  P  I  N  P  P  I  F  P  P  T  E  R  N  E  I  V  A  Y  L  L
CGTATGTTTCTCCCACTTGGCACGCATTACGCGCTGTCATTTTTCAGCAGATGAATATGCAGCCTATTAATCCGCCAATTTTTCCACCGACTGAAAGGAATGAAATTGTTGCGTATCTAT
       850       860       870       880       890       900       910       920       930       940       950       960

 L  V  A  S  L  A  D  V  Y  A  A  L  R  P  D  F  R  M  N  G  V  V  A  P  V  G  Q  I  N  R  A  L  V  L  A  A  Y  H  *
TATTAGTAGCTTCTTTAGCTGATGTGTATGCGGCTTTGAGGCCAGATTTCAGAATGAATGGTGTTGTTGCGCCAGTAGGCCAGATCAACAGAGCTCTTGTGCTAGCAGCCTACCACTAGT
       970       980       990      1000      1010      1020      1030      1040      1050      1060      1070      1080

GGCTGCGGTGTTGCACGGTCACCGCTTTCATTAGTGTCGCGTCGGTTCTTATGCTGATAAAGTACGCATAAGTAATACGTCAATACCGAATACACTTAC
      1090      1100      1110      1120      1130      1140      1150      1160      1170
```

Fig 1

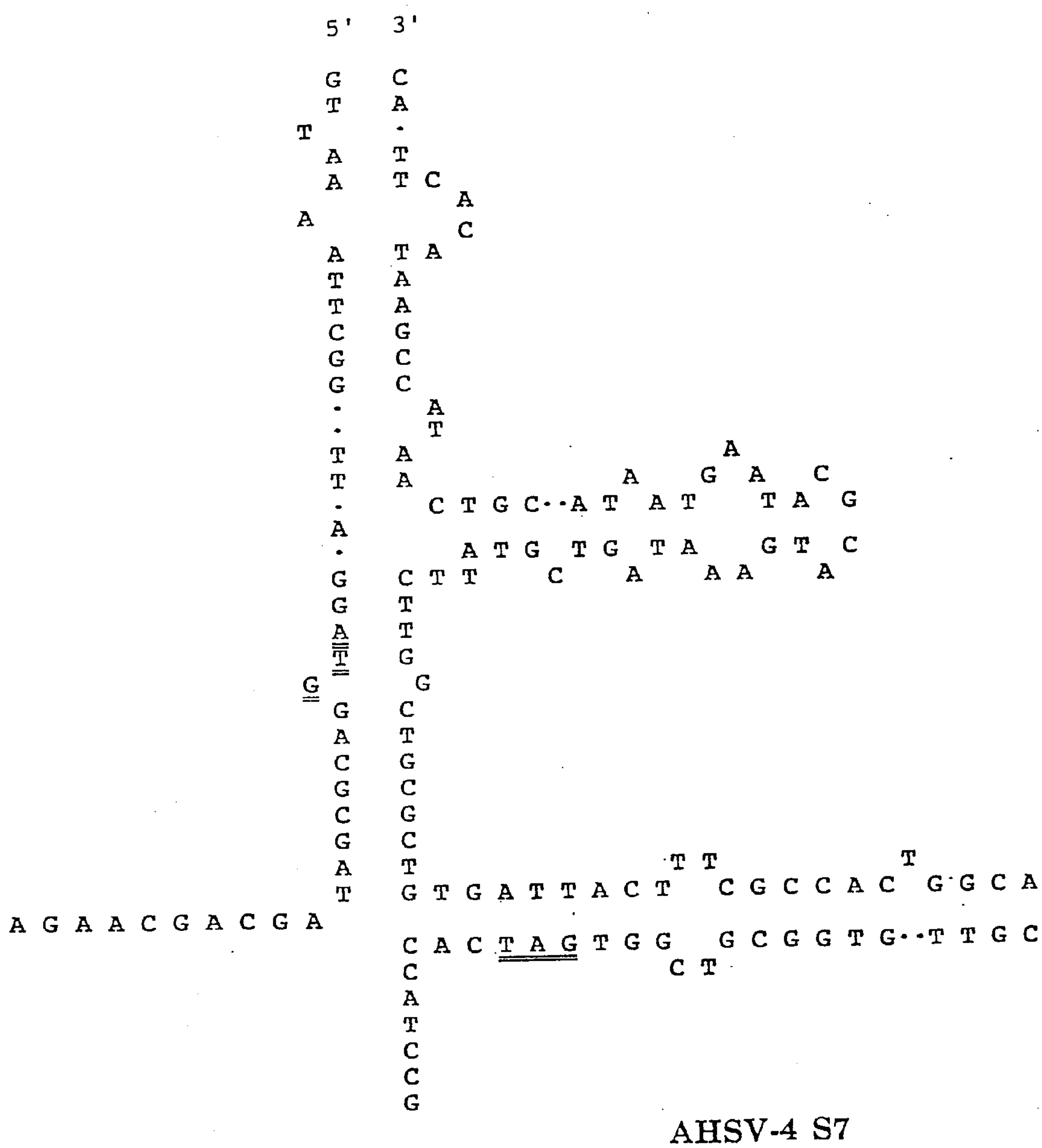


AHSV-4 S7

Fig 2a

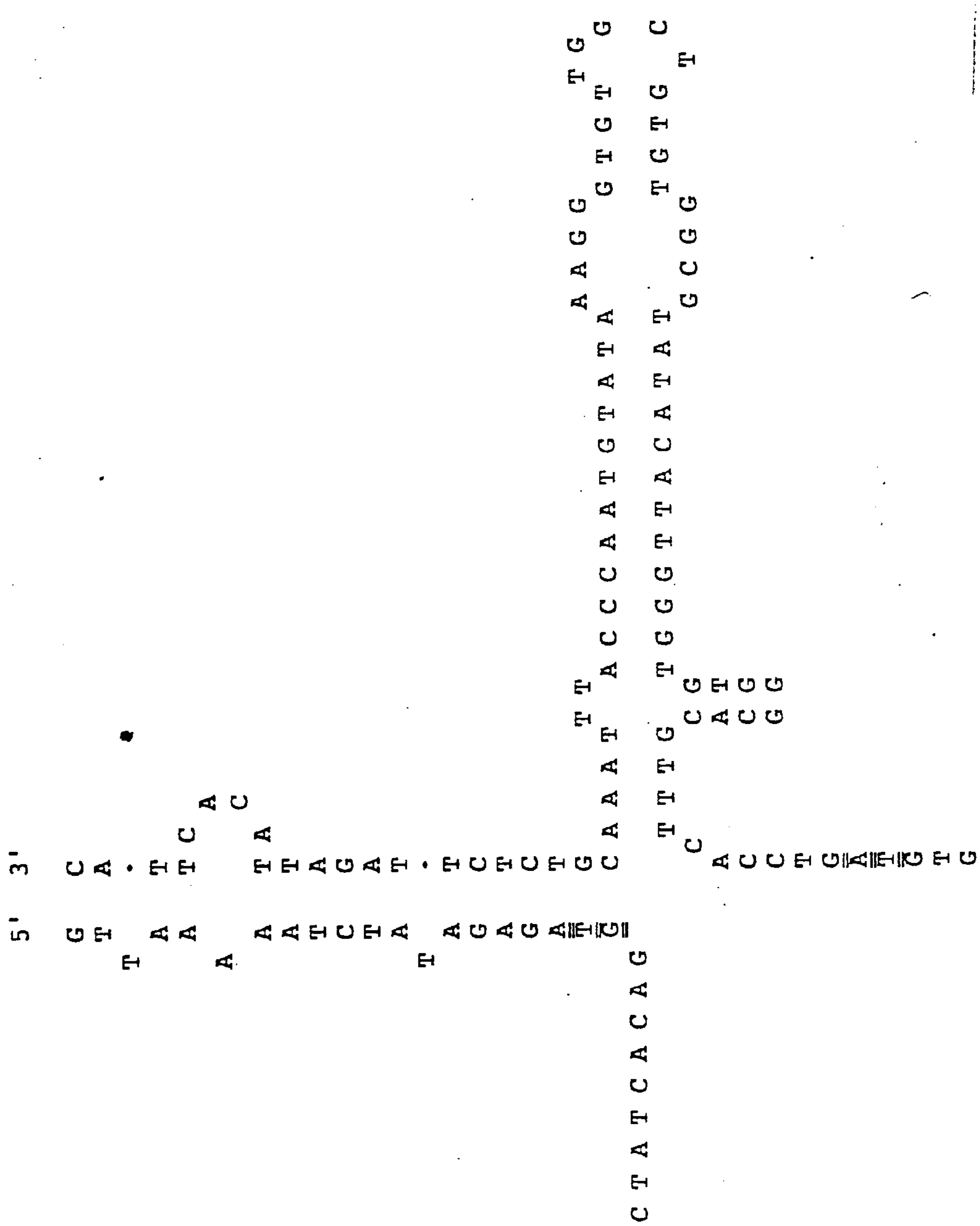

BTV-10 S7

Fig 2b

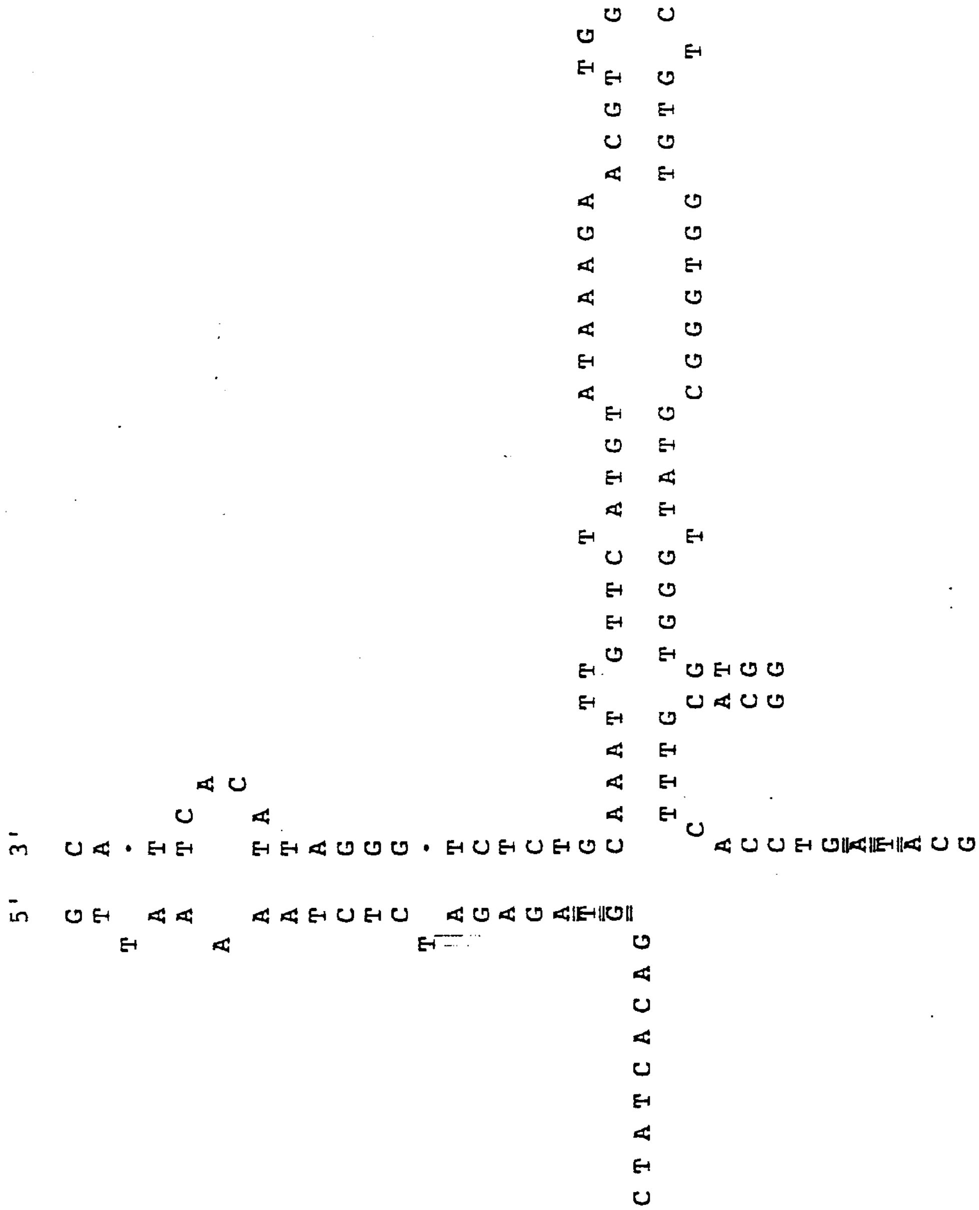

BTV-13 S7

Fig 2c

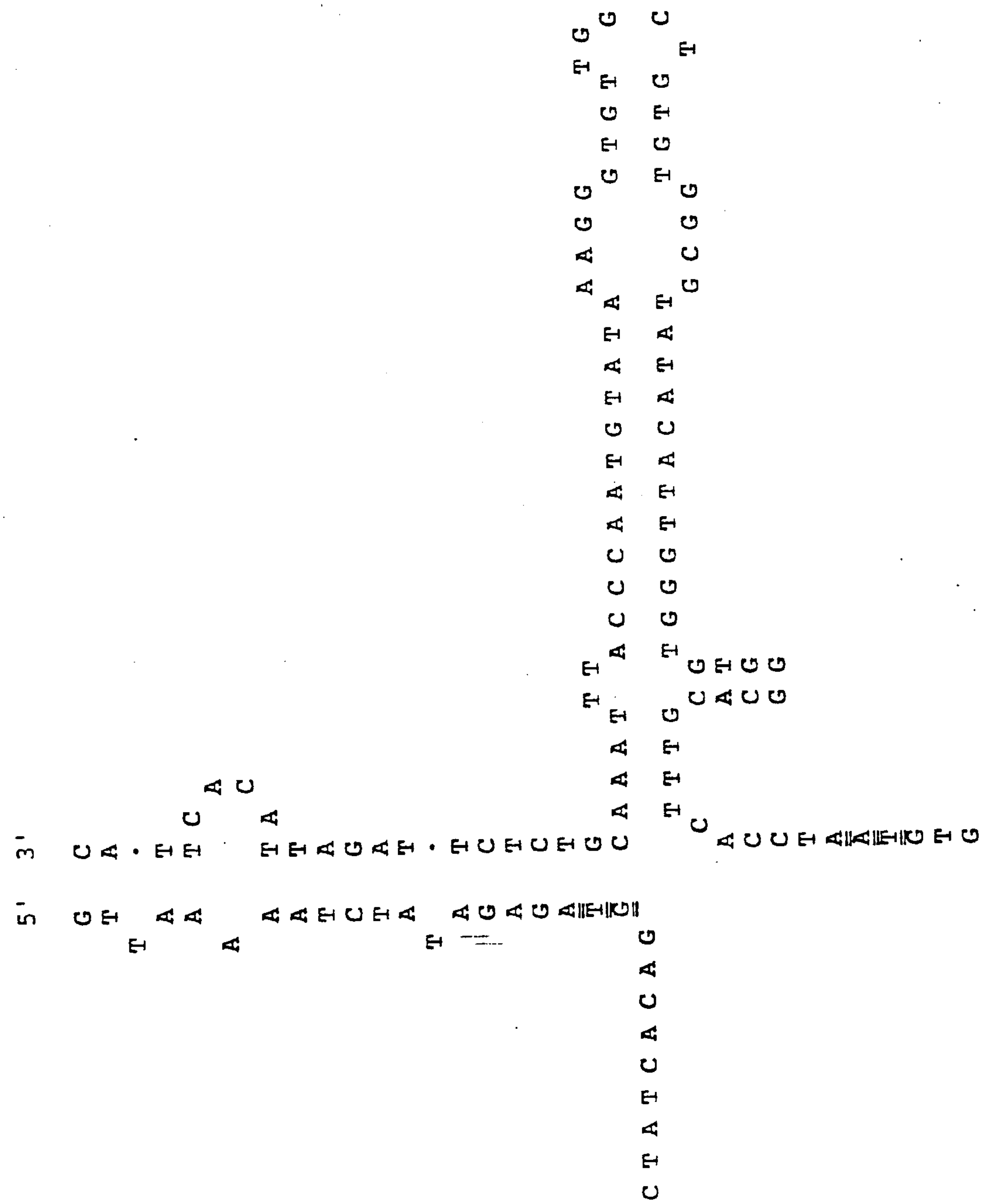

BTV-17 S7

Fig 2d

```
BTV-10   MDTIAARALT VMRACATLQE ARIVLEANVM EILGIAINRY NGLTLRGVTM RPTSLAQRNE  60
ASHV-4   MDAIAARALS VVRACVTVTD ARVSLDPGVM ETLGIAINRY NGLTNHSVSM RPQTQAERNE
         ** ******  * *** *    **  *   ** * ******** ****   * * **   * ***

BTV-10   MFFMCLDMML SAAGINVGPI SPDYTQHMAT IGVLATPEIP FTTEAANEIA RVTGETSTWG  120
AHSV-4   MFFMCTDMVL AALNVQIGNI SPDYDQALAT VGALATTEIP YNVQAMNDIV RITGQMQTFG
         ***** ** *  *     * * **** *  **  * *** ***     * * *  * **   * *

BTV-10   PAR---QPYG FFLETEETFQ PGRWFMRAAQ AVTAVVCGPD MIQVSLNAGA RGDVQQIFQG  180
AHSV-4   PSKVQTGPYA GAVEVQ---Q SGRYYVPQGR TRGGYI-NSN IAEVCMDAGA AGQVNALLAP
         *      **     *     *  **                     *   ***  * *

BTV-10   RN-DPMMIYL VWRRIENFAM AQGNSQQTQA GVT---VSVG GVDMRAGRII AWDGQAALHV  240
AHSV-4   RRGDAVMIYF VWRPLRIFCD PQGASLESAP GAPGTFVTVD GVNVAAGDVV AWNTIAPVNV
         *  *  ***  ***    *    ** *     *     * *  **   **    **   *   *

BTV-10   HNPTQQNAMV QIQVVFYISM DKTLNQYPAL TAEIFNVYSF RDHTWHGLRT AILNRTTLPN  300
AHSV-4   GNPGARRSIL QFEVLWYTSL DRSLDTVPEL APTLTRCYAY VSPTWHALRA VIFQQMNMQP
          **        *  *  * *  *  *   * *        *      *** **  *

BTV-10   MLPPIFPPND RDSILT-LLL LSTLADVYTV LRPEFAIHGV NPMPGPLTRA IARAAYV     357
AHSV-4   INPPIFPPTE RNEIVAYLLL VASLADVYAA LRPDFKVNGV VAPVGQINRA LVLAAYH
           ******   *  *   ***    *****  *** *   **     *   **    ***
```

Fig. 3

A

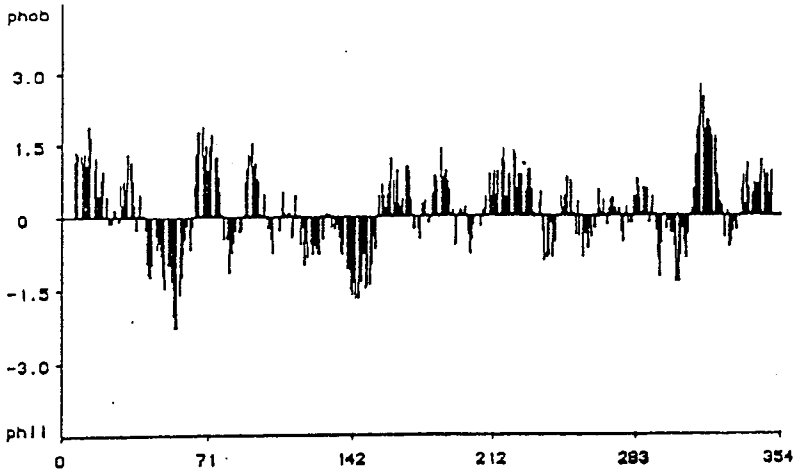
phob
3.0
1.5
0
-1.5
-3.0
phil
0
71
142
212
283
354

B

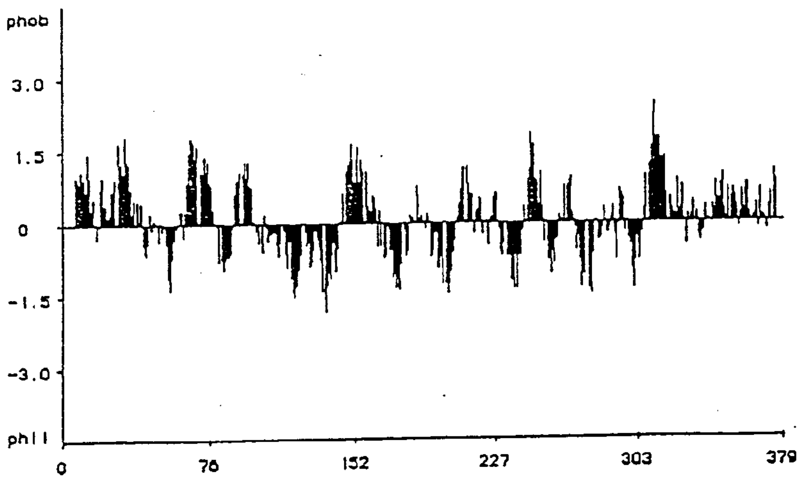
phob
3.0
1.5
0
-1.5
-3.0
phil
0
76
152
227
303
379

Fig 4

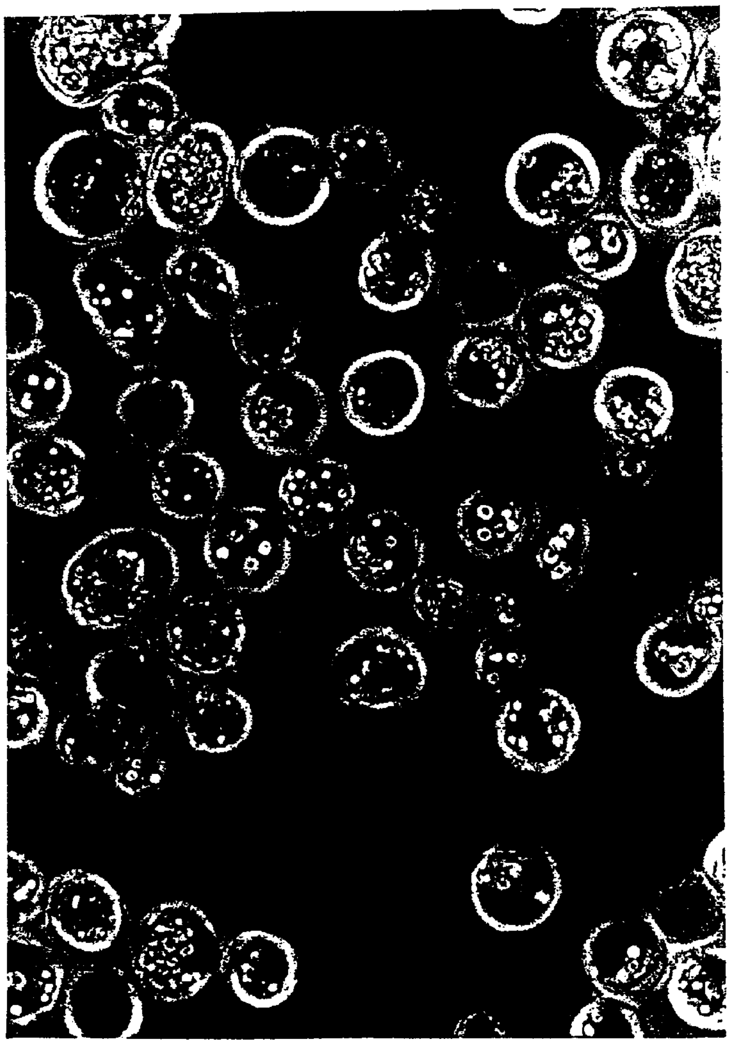

AcNPV infected Sf cells x 40

Fig 5

#99

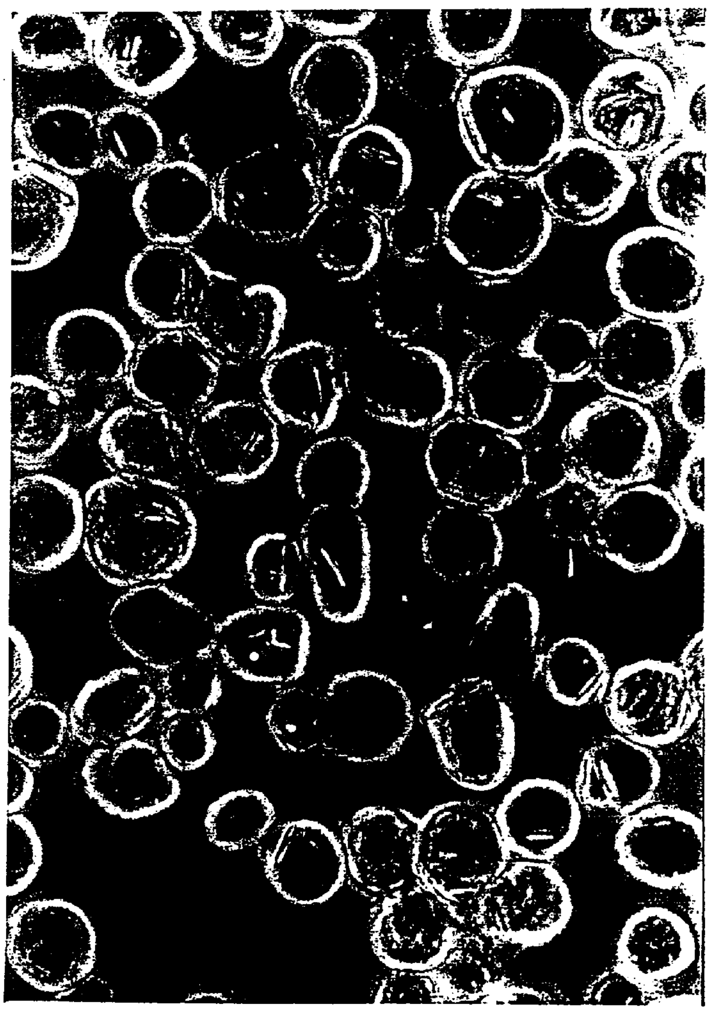

AHSV-VP7 infected Sf cells x40.

Fig 6

#9

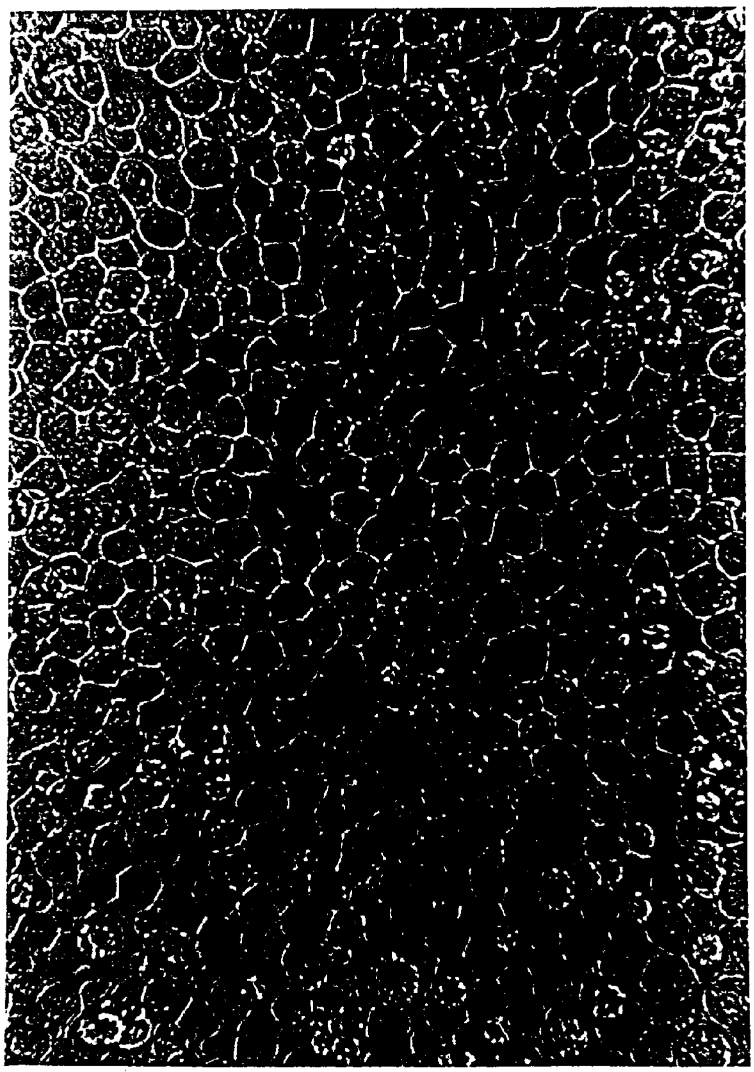

Mock infected Sf cells x 20

Fig 7

APPENDIX A - Figure 1

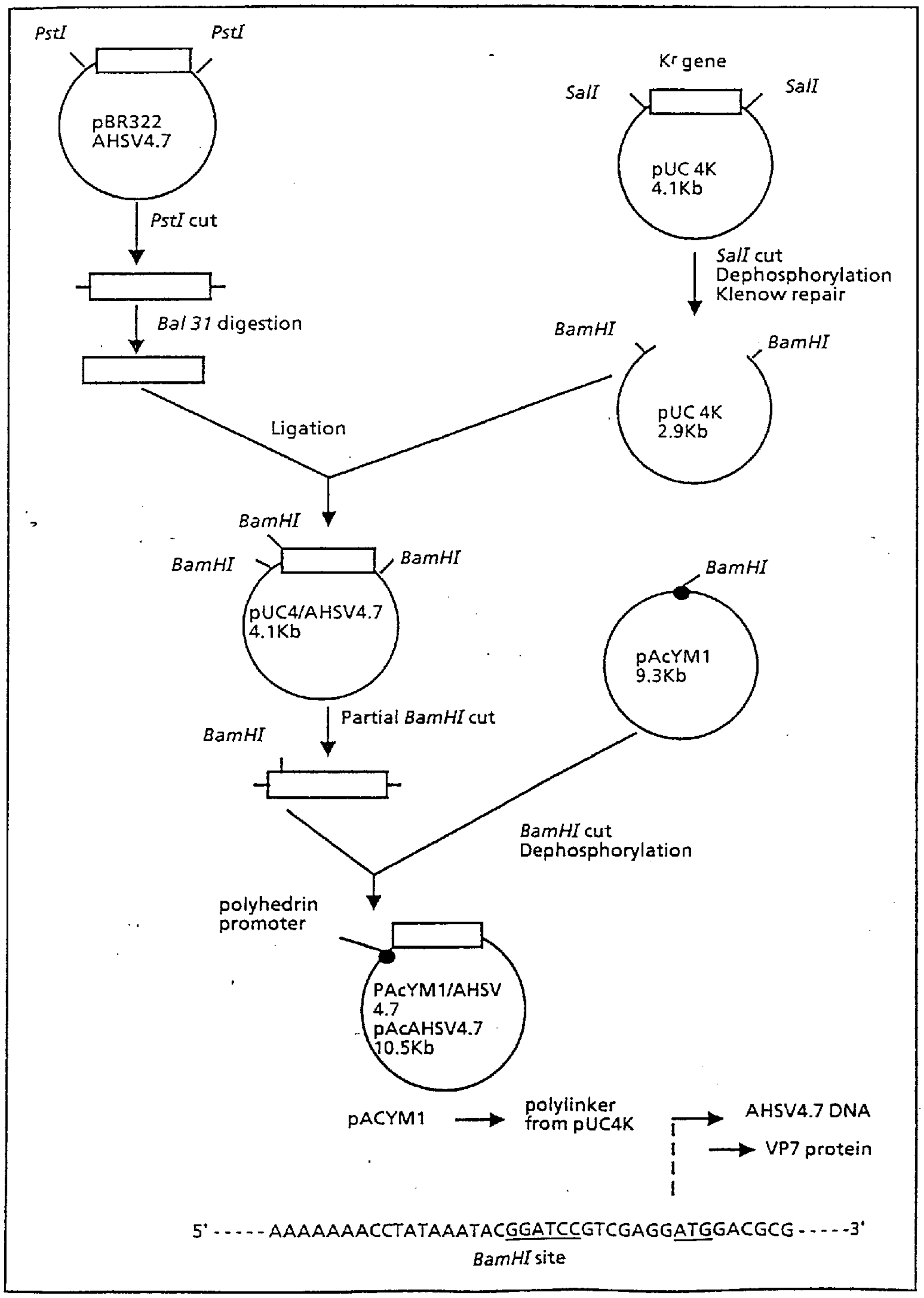

# APPENDIX A - Figure 2

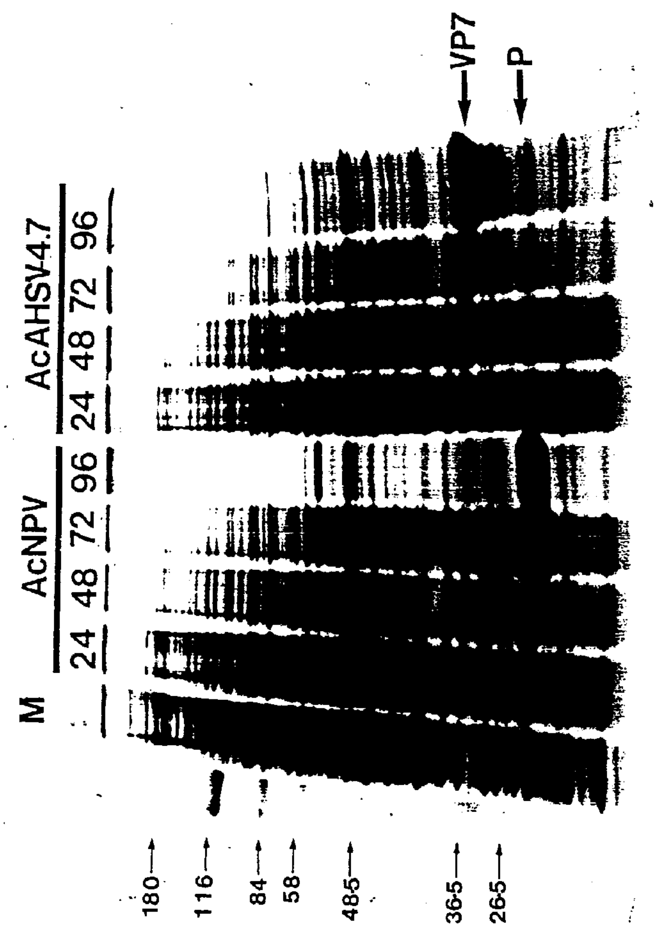

APPENDIX A - Figure 3A

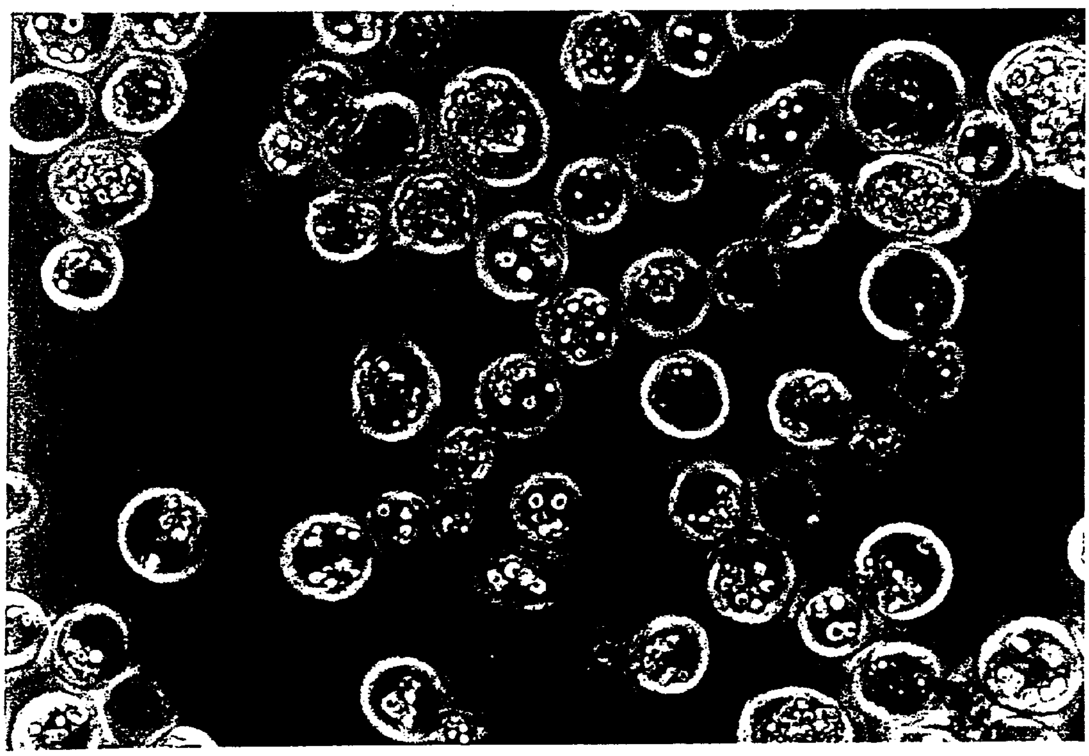

A

APPENDIX A - Figure 3B

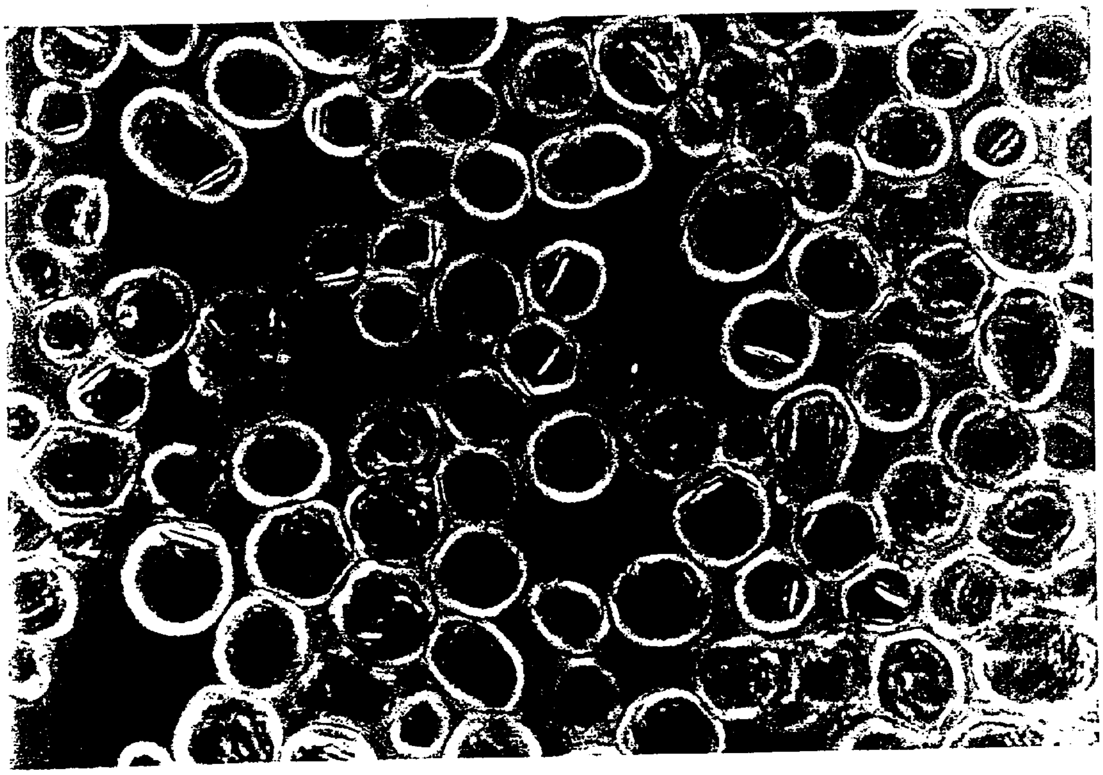

B

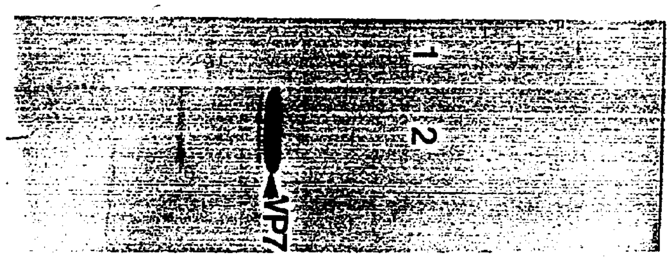


Figure 4

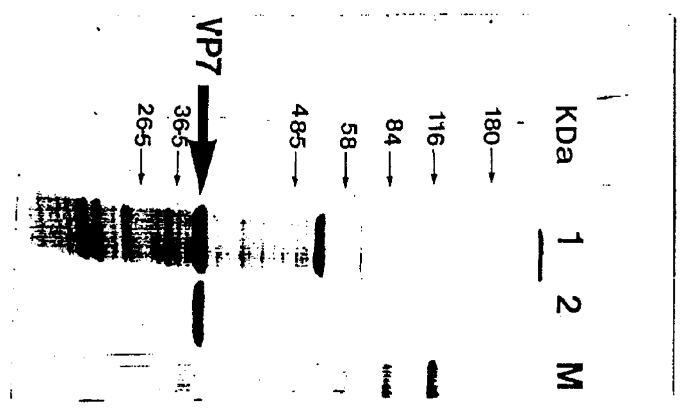


Figure 5

# APPENDIX A - Figure 6

A

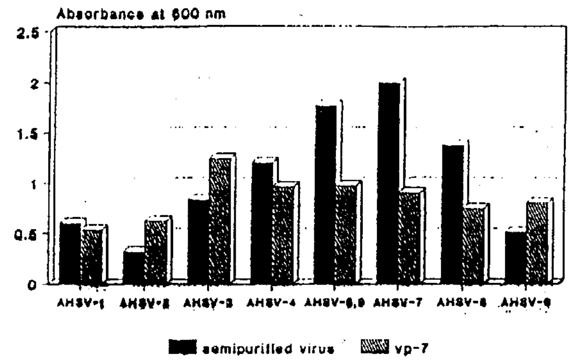


B

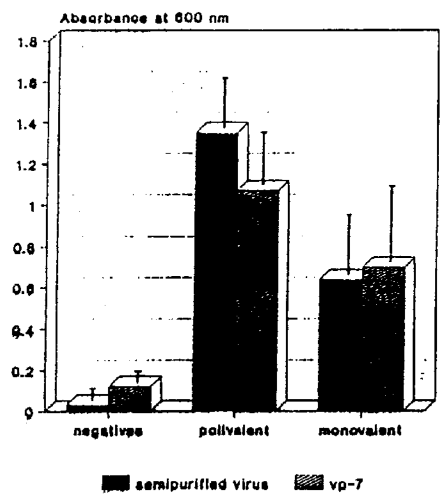


Fig.6 Ability of antibodies to AHSV to bind to VP7 compared with semipurified virus in an indirect ELISA assay.

A: sera from horses inoculated with eight different serotypes.

B: sera from horses of the following groups: negative, vaccinated with a polivalent vaccine and vaccinated with monovalent vaccine (serotype 4).

Bars represent the average value of 10,10 and 34 sera respectively. The bracketed lines represent the standard deviation.

APPENDIX A - Figure 7